(19) Europäisches Patentamt
European Patent Office
Office européen des brevets

(11) **EP 3 517 139 B1**

(12) **EUROPÄISCHE PATENTSCHRIFT**

(45) Veröffentlichungstag und Bekanntmachung des Hinweises auf die Patenterteilung:
**04.08.2021 Patentblatt 2021/31**

(51) Int Cl.:
*A61L 2/10* (2006.01)     *A61L 2/24* (2006.01)
*B08B 15/02* (2006.01)

(21) Anmeldenummer: **19000028.1**

(22) Anmeldetag: **16.01.2019**

(54) **LABORGERÄT MIT UV-BESTRAHLUNGSVORRICHTUNG SOWIE DESINFEKTIONSVERFAHREN FÜR EIN LABORGERÄT**

LABORATORY DEVICE WITH UV RADIATION UNIT AND DISINFECTION METHOD FOR A LABORATORY DEVICE

APPAREIL DE LABORATOIRE POURVU DE DISPOSITIF D'IRRADIATION UV AINSI QUE PROCÉDÉ DE DÉSINFECTION POUR UN APPAREIL DE LABORATOIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.01.2018 DE 102018000575**

(43) Veröffentlichungstag der Anmeldung:
**31.07.2019 Patentblatt 2019/31**

(73) Patentinhaber: **Thermo Electron LED GmbH**
**63505 Langenselbold (DE)**

(72) Erfinder:
• **Phillips, David Scott**
**Arnold, MD 21012 (US)**
• **Rupp, Oliver**
**63505 Langenselbold (DE)**

(74) Vertreter: **Tomerius, Isabel**
**Lang & Tomerius**
**Patentanwaltspartnerschaft mbB**
**Rosa-Bavarese-Strasse 5**
**80639 München (DE)**

(56) Entgegenhaltungen:
EP-A1- 3 269 798        GB-A- 2 551 349
US-A1- 2008 199 353     US-A1- 2009 218 512
US-A1- 2015 060 696

**Beschreibung**

Die Erfindung betrifft ein Laborgerät mit einer UV-Bestrahlungsvorrichtung, die wenigstens einen UV-Strahler zur Durchführung eines Desinfektionsvorgangs umfasst. UV-Strahlung, vor allem UV-C-Strahlung, bevorzugt mit einer Wellenlänge von 254 nm, ist geeignet, Bakterien, Viren, Sporen und Hefen abzutöten. UV-Strahlung wird daher häufig zur Desinfektion von Arbeits- oder Lagerräumen von Laborgeräten verwendet, in denen biologische oder mikrobiologische Proben bearbeitet oder gelagert werden.

Über welchen Zeitraum eine Bestrahlung mit UV-Strahlung stattfinden muss, um eine ausreichende Desinfektion zu erreichen, hängt von der Größe des zu bestrahlenden Raums, Art und Umfang der Desinfektion und nicht zuletzt von dem verwendeten UV-Strahler selbst ab. Ein geeigneter Bestrahlungs-Zeitraum wird in der Regel vom Benutzer des Laborgeräts entsprechend den Gegebenheiten vorab bestimmt und dann in eine Steuervorrichtung eingegeben, welche die UV-Bestrahlungsvorrichtung entsprechend den Vorgaben steuert. Es ist allerdings auch möglich, dass ein geeigneter Bestrahlungs-Zeitraum vorab vom Hersteller des Laborgeräts in der Steuervorrichtung vorgegeben ist, der gegebenenfalls vom Benutzer korrigiert werden kann.

Wie bei anderen Leuchtmitteln auch nimmt die Intensität der Strahlung von UV-Strahlern mit zunehmender Betriebsdauer des Strahlers kontinuierlich ab. Damit besteht die Gefahr, dass mit zunehmender Lebensdauer des UV-Strahlers die Strahlungsmenge nicht mehr ausreicht, um das gewünschte Desinfektionsergebnis zu erzielen. Um dies zu vermeiden, werden in der Praxis Desinfektionsvorgänge häufig vorsorglich erheblich länger ausgeführt, als dies tatsächlich für eine ausreichende Desinfektion erforderlich wäre. Beispielsweise werden Desinfektionsvorgänge oft die gesamte Nacht hindurch laufen gelassen. Dies zieht nicht nur einen erheblich erhöhten Energieverbrauch nach sich, sondern die starke Belastung mit UV-Strahlen schädigt auch die Komponenten des Laborgeräts, welche der Strahlung ausgesetzt sind. Eine andere Möglichkeit, eine unzureichende Desinfektion zu verhindern, besteht im frühzeitigen Austausch des UV-Strahlers. Die Hersteller von UV-Strahlern geben in der Regel Benutzungsdauern bekannt, ab denen die Intensität der UV-Strahlung sich um ein bestimmtes Maß verringert und/oder eine maximale Benutzungsdauer, nach der der UV-Strahler nicht weiter benutzt werden sollte. Ein verfrühter Austausch des UV-Strahlers stellt jedoch nicht nur eine Ressourcenverschwendung dar, sondern verursacht auch erhebliche Kosten. Grundsätzlich ist auch denkbar, dass der Benutzer des Laborgeräts die benötigte Desinfektionszeit mit zunehmender Betriebsdauer des eingesetzten UV-Strahlers immer wieder überprüft und angepasst. Dies ist für den Benutzer jedoch mit erheblichem Aufwand verbunden und daher unerwünscht.

Die US 2009/218512 A1 beschreibt ein Desinfektionsgerät für Computertastaturen unter Verwendung von UV-C-Strahlung. Die Dauer der UV-C-Bestrahlung kann dabei abhängig von der Bestrahlungsintensität, die mittels eines Lichtsensors ermittelt wird, auf unterschiedliche Längen eingestellt werden. Steuervorrichtungen zur Steuerung von UV-Strahlern sind beschrieben in GB 2551349 A, US 2015/0060696 A1 und US 2008/0199353 A1. Die EP3269798 A1 beschreibt einen Inkubator mit einer UV-Lampe zur Desinfektion.

Aufgabe der Erfindung ist es entsprechend, ein Laborgerät und ein Verfahren für dessen Betrieb anzugeben, bei denen die oben angesprochenen Probleme nicht auftreten und welche auch bei einer längeren Betriebsdauer des wenigstens einen UV-Strahlers in einer UV Bestrahlungsvorrichtung eine ausreichende Desinfektion sicherstellen, ohne dabei die Desinfektionszeit unnötig zu verlängern oder dem Benutzer des Laborgeräts zusätzlichen Aufwand zu verursachen.

Die Lösung dieser Aufgabe gelingt mit dem Laborgerät nach Anspruch 1 sowie dem Verfahren nach Anspruch 9. Bevorzugte Weiterbildungen und Verfahrensvarianten sind in den jeweiligen Unteransprüchen beschrieben.

In einem ersten Aspekt betrifft die Erfindung also ein Laborgerät, in dem biologische oder mikrobiologische Proben bearbeitet oder gelagert werden und das eine UV-Bestrahlungsvorrichtung mit mindestens einem UV-Strahler zur Durchführung eines Desinfektionsvorgangs umfasst sowie eine Steuervorrichtung zur Steuerung des UV-Strahlers aufweist. Die Steuervorrichtung ist ausgebildet, eine Zeitdauer vorzugeben, während welcher der UV-Strahler während des Desinfektionsvorgangs betrieben wird. Erfindungsgemäß ist die Steuervorrichtung weiter ausgebildet, die Zeitdauer unter Verwendung einer in der Steuervorrichtung hinterlegten Steuerfunktion in Abhängigkeit von der Betriebsdauer des UV-Strahlers so vorzugeben, dass die Zeitdauer mit zunehmender Betriebsdauer zunimmt.

Im erfindungsgemäßen Laborgerät ist die Steuervorrichtung, welche die UV-Bestrahlungsvorrichtung während des Desinfektionsvorgangs steuert, dazu ausgebildet, die Zeitdauer des Desinfektionsvorgangs der Betriebsdauer des mindestens einen UV-Strahlers in der Bestrahlungsvorrichtung automatisch anzupassen. Je länger der mindestens eine UV-Strahler bereits in der UV-Bestrahlungsvorrichtung betrieben wurde, desto mehr wird die Zeitdauer des Desinfektionsvorgangs erhöht, um die mit längerer Betriebsdauer einhergehende Reduzierung der vom UV-Strahler emittierten Strahlungsmenge zu kompensieren. In welcher Weise diese Kompensation erfolgt, hängt von der Art des verwendeten UV-Strahlers ab. Wie bereits erwähnt, geben die Hersteller von UV-Strahlern zu jedem Lampentyp üblicherweise die Abnahme der Strahlungsmenge bzw. der Bestrahlungsstärke mit der Lebensdauer an, welche hier der Betriebsdauer des UV-Strahlers in der UV-Bestrahlungsvorrichtung gleichgesetzt wird. Diese Herstellerangaben zur abnehmenden Bestrahlungsstärke können die Grundlage für die Ermittlung der Verlängerung der Zeitdauer des Desinfektionsvorgangs bilden. Falls erforderlich, kann der Zusammenhang der Abnahme der Strahlungsmenge bzw. Bestrahlungsstärke mit zunehmender Lebensdauer auch vorab vom Hersteller des Laborgeräts in an sich bekannter Weise ermittelt werden.

Der Kompensation der abnehmenden Bestrahlungsstärke mit zunehmender Betriebsdauer des mindestens einen UV-Strahlers liegt zu Grunde, dass die Bestrahlung (Strahlungsenergie/Fläche) das Produkt von Bestrahlungsstärke und Bestrahlungszeit ist. Nimmt die Bestrahlungsstärke also ab, kann dies durch Verlängerung der Bestrahlungszeit in einfacher Weise ausgeglichen werden, um im Laborgerät eine gleichbleibende Bestrahlung zu gewährleisten. Es muss daher für den verwendeten UV-Strahler lediglich ermittelt werden, um welchen Faktor bzw. Prozentsatz die Bestrahlungsstärke für verschiedene Betriebsdauern von einer Referenz-Bestrahlungsstärke abweicht, um die Zeitdauer entsprechend der festgestellten Abweichung zu korrigieren - konkret beispielsweise, die Bestrahlungszeit mit dem Reziprokwert des festgestellten Abweichungsfaktors zu multiplizieren. Die Referenz-Bestrahlungsstärke kann beispielsweise die Nenn-Bestrahlungsstärke des UV-Strahlers sein, welche üblicherweise vom Hersteller des UV-Strahlers in dessen Produktdatenblatt bekannt gegeben wird. Entsprechend dem ermittelten Faktor oder Prozentsatz wird bei der zugehörigen Betriebsdauer die Bestrahlungszeit so verlängert, dass die resultierende Bestrahlung im Wesentlichen konstant bleibt. Anstelle der Nenn-Bestrahlungsstärke als Referenzwert kann alternativ auch eine Referenz-Bestrahlungsstärke bei einer bestimmten Betriebsdauer des UV-Strahlers verwendet werden, die dann als 100 % definiert wird. Dies kann die Bestrahlungsstärke des UV-Strahlers bei erstmaliger Inbetriebnahme sein. Zu berücksichtigen ist dabei allerdings, dass einige UV-Strahler erst eine gewisse Einlaufzeit benötigen, bis sie die beabsichtigte Bestrahlungsstärke erreicht haben. Es ist daher bevorzugt, den Referenzwert erst nach Ablauf der Einlaufzeit festzusetzen. Häufig lassen sich die benötigten Werte der Bestrahlungsstärke aus vom Hersteller bereitgestellten Diagrammen entnehmen, welche die Bestrahlungsstärke gegen die Lebensdauer des UV-Strahlers aufzeichnen.

Die Korrektur der Zeitdauer in Abhängigkeit von der Betriebsdauer des mindestens einen UV-Strahlers durch die Steuervorrichtung kann kontinuierlich oder schrittweise erfolgen, indem in an sich bekannter Weise aus den ermittelten Korrekturwerten eine Korrekturfunktion erstellt und in der Steuervorrichtung hinterlegt wird. Im Fall der schrittweisen Korrektur erfolgt die Verlängerung der Zeitdauer jeweils nur, wenn bestimmte Schwellenwerte der Betriebsdauer des UV-Strahlers erreicht sind. Die Verlängerung der Zeitdauer des Desinfektionsvorgangs erfolgt entsprechend stufenweise nach dem jeweiligen Erreichen eines Schwellenwertes. Daraus ergibt sich, dass es erfindungsgemäß nicht erforderlich ist, dass die Bestrahlung mit zunehmender Betriebsdauer des mindestens einen UV-Strahlers immer exakt der ursprünglich vorgegebenen Bestrahlung entsprechen muss, sondern vielmehr Abweichungen zulässig sind, solange dadurch das gewünschte Desinfektionsergebnis nicht infrage gestellt wird. Toleranzbereiche sind nicht nur bei einer schrittweisen Anpassung der Zeitdauer, sondern auch im Falle einer kontinuierlichen Anpassung möglich. Wie groß die Toleranzbereiche sein können, hängt von der konkreten Ausgestaltung des jeweiligen Laborgeräts ab. Grundsätzlich können jedoch beispielsweise Abweichungen von bis zu 10 % von der ursprünglich vorgegebenen Bestrahlung zumindest zeitweilig auftreten, wobei die Abweichungen vorzugsweise weniger als 5 % und insbesondere weniger als 2 % betragen.

Während der Betriebszeit des mindestens einen UV-Strahlers, in welcher dieser noch mit der gewünschten Bestrahlungsstärke arbeitet und noch keine Verschlechterung stattgefunden hat, ist es nicht notwendig, die Zeitdauer des Desinfektionsvorgangs gegenüber der ursprünglich in der Steuervorrichtung hinterlegten Zeitdauer zu verlängern. Aus diesem Grund ist es erfindungsgemäß bevorzugt, mit der Verlängerung der Zeitdauer für den Desinfektionsvorgang erst dann zu beginnen, wenn eine gewisse Verschlechterung des UV-Strahlers tatsächlich bereits stattgefunden hat oder eine solche Verschlechterung zu erwarten ist. Dieser Zeitraum wird nachfolgend als Sollbetriebsdauer bezeichnet. In der Regel kann er den vom Hersteller verfügbaren Lampenspezifikationen entnommen werden oder lässt sich durch Tests leicht ermitteln. Bei der Sollbetriebsdauer handelt es sich insbesondere um den Zeitraum vom ersten Starten des UV-Strahlers in der UV-Bestrahlungsvorrichtung bis zu dem Zeitpunkt, an dem der UV-Strahler UV-Strahlung mit einer Bestrahlungsstärke emittiert, die gerade noch einer Soll-Bestrahlungsstärke und insbesondere der Nenn-Bestrahlungsstärke entspricht. Bis zum Erreichen dieser Sollbetriebsdauer wird der mindestens eine UV-Strahler der UV-Bestrahlungsvorrichtung während des Desinfektionsvorgangs mit der ursprünglich in der Steuervorrichtung hinterlegten Zeitdauer betrieben. Erst ab dieser Sollbetriebsdauer nimmt die Steuervorrichtung dann die Verlängerung der Zeitdauer entsprechend dem vorstehend Dargelegten vor.

Mit der erfindungsgemäß von der Steuervorrichtung vorgenommenen Verlängerung der Zeitdauer in Abhängigkeit von der Betriebsdauer des mindestens einen UV-Strahlers in der UV-Bestrahlungsvorrichtung können Desinfektionsvorgänge ohne vorzeitigen Austausch der UV-Strahler und ohne zusätzlichen Aufwand für den Benutzer bei Vermeidung unnötigen Energieverbrauchs sicher durchgeführt werden. Die Lebensdauer der UV-Strahler kann erheblich besser als im Stand der Technik ausgeschöpft werden. Allerdings tritt auch bei Anwendung der Erfindung irgendwann der Zeitpunkt ein, an dem die Bestrahlungsstärke des UV-Strahlers so stark abgenommen hat, dass eine zuverlässige Desinfektion nicht mehr gewährleistet ist. Nach Erreichen dieses Zeitpunkts sollte der UV-Strahler also ausgetauscht werden. Um dies sicherzustellen, ist es bevorzugt, in der Steuervorrichtung eine maximale Betriebsdauer zu hinterlegen. Diese entspricht insbesondere der vom Hersteller des UV-Strahlers definierten maximalen Lebensdauer. Stellt die Steuervorrichtung das Erreichen der maximalen Betriebsdauer fest, kann sie beispielsweise veranlassen, dass dem Benutzer eine entsprechende Warnung ausgegeben wird. Diese kann in einem optischen und/oder akustischen Signal bestehen, beispielsweise einem Hinweis in einem Display des Laborgeräts.

Bevorzugt ist es weiterhin, dass die Steuervorrichtung ausgebildet ist, die Verlängerung der Zeitdauer im Zeitraum zwischen der Sollbetriebsdauer und der maximalen Betriebsdauer vorzunehmen. Nach dem Erreichen der maximalen Betriebsdauer findet somit keine (weitere) Verlängerung der Zeitdauer des Desinfektionsvorgangs statt. Dies hat den Hintergrund, dass eine noch weitere Verlängerung der Zeitdauer eine zuverlässige Desinfektion wegen der bereits deutlichen Verschlechterung des UV-Strahlers gegebenenfalls ohnehin nicht mehr gewährleisten kann und daher auch nicht mehr sinnvoll ist. Zweckmäßig ist es jedoch, auch nach Überschreiten der maximalen Betriebsdauer eine Zeitverlängerung entsprechend derjenigen bei der maximalen Betriebsdauer weiter beizubehalten. Entsprechend gliedert sich ein bevorzugter Zyklus eines UV-Strahlers in einem erfindungsgemäßen Laborgerät bzw. nach dem erfindungsgemäßen Verfahren in drei Phasen mit einer ersten Phase, die bis zum Erreichen der Sollbetriebsdauer reicht und bei der der UV-Strahler während des Desinfektionsvorgangs mit der ursprünglich vorgegebenen Zeitdauer betrieben wird, einer zweiten Phase, die von der Sollbetriebsdauer bis zur maximalen Betriebsdauer reicht und in der die Zeitdauer in Abhängigkeit von der Betriebsdauer des UV-Strahlers in der UV-Bestrahlungsvorrichtung verlängert wird, sowie einer dritten Phase ab der maximalen Betriebsdauer, die für einen kurzen Zeitraum einen Weiterbetrieb des Laborgeräts gewährleisten soll, bis der UV-Strahler durch einen neuen UV-Strahler ersetzt worden ist.

Grundsätzlich ist es möglich, die Verlängerung der Zeitdauer des Desinfektionsbetriebs in Abhängigkeit von der Betriebsdauer des mindestens einen UV-Strahlers genau entsprechend der für die jeweilige Betriebsdauer ermittelten Abnahme der Bestrahlungsstärke durchzuführen. Wie bereits mehrfach erwähnt, sind für viele UV-Strahler derartige Diagramme, die den Verlauf der Abnahme der Bestrahlungsstärke über die Lebensdauer des Strahlers durchgängig wiedergeben, verfügbar. Es ist also möglich, diese genauen Werte für die Korrektur der Zeitdauer über die Betriebsdauer einzusetzen. Allerdings ist in den meisten Fällen eine solche Genauigkeit bei der Durchführung eines Desinfektionsvorgangs gar nicht erforderlich und würde nur unnötigen Rechenaufwand in der Steuervorrichtung verursachen. Bevorzugt ist es erfindungsgemäß daher, bei der Berechnung der Verlängerung der Zeitdauer in Abhängigkeit von der Betriebsdauer eine Linearfunktion zu Grunde zu legen. Diese Linearfunktion kann für den verwendeten UV-Strahler aus den vom Hersteller verfügbar gemachten Spezifikationen oder selbst ermittelten Werten durch geeignete Näherung bestimmt werden. Eine Möglichkeit besteht in der Anwendung einer linearen Regression, also durch Ermittlung einer Ausgleichsgerade aus den zur Verfügung stehenden Werten. Statt die exakten Werte zu verwenden, wird also der Korrekturfaktor zur Berechnung der Verlängerung der Zeitdauer für die jeweilige Betriebsdauer aus der Ausgleichsgerade entnommen oder anhand einer für diese hinterlegten Funktion errechnet.

Die Linearfunktion kann auch lediglich als Verbindung von zwei Punkten definiert sein. Bei diesen beiden Punkten kann es sich beispielsweise um die Bestrahlungsstärken bei der Sollbetriebsdauer einerseits und der maximalen Betriebsdauer andererseits handeln. Die Bestrahlungsstärke bei der Sollbetriebsdauer wird dann zweckmäßig als 100 % festgelegt, und die Bestrahlungsstärke bei der maximalen Betriebsdauer wird als x % (< 100 %) ermittelt. Zwischen beiden Eckpunkten wird eine lineare Abnahme der Bestrahlungsstärke angenommen. Die Abnahme der Bestrahlungsstärke für diejenigen Betriebsdauern, die im Bereich zwischen Sollbetriebsdauer und maximaler Betriebsdauer liegen, ergibt sich somit jeweils aus der Geraden, welche die Werte für die Sollbetriebsdauer und die maximale Betriebsdauer einschließt bzw. aus der Linearfunktion, durch welche diese Gerade definiert ist. Aus dieser Linearfunktion ergibt sich somit auch der Korrekturfaktor für die Verlängerung der Zeitdauer im Zeitraum zwischen Sollbetriebsdauer und maximaler Betriebsdauer.

Wird die Bestrahlungsstärke bei der Sollbetriebsdauer mit $I_S$ bezeichnet und die Bestrahlungsstärke bei der maximalen Betriebsdauer mit $I_{Max}$, ergibt sich unter der obigen Annahme einer linearen Abnahme der Bestrahlungsstärke im Bereich einer Betriebsdauer zwischen der Sollbetriebsdauer $d_S$ und der maximalen Betriebsdauer $d_{Max}$ für eine beliebige Betriebsdauer $d_X$ in diesem Bereich der Korrekturfaktor für die Verlängerung der Zeitdauer als

$$\left(\frac{I_S - I_{Max}}{I_S}\right)(d_X - d_s)/(d_{Max} - d_s)$$

Solange die Sollbetriebsdauer noch nicht erreicht ist, wird jeder Desinfektionsvorgang mit der in der Steuervorrichtung hinterlegten (Standard-) Zeitdauer $t_S$ durchgeführt. Im Betriebszeitraum der Sollbetriebsdauer $d_S$ und der maximalen Betriebsdauer $d_{Max}$ erfolgt dagegen eine Verlängerung der Zeitdauer um den oben beschriebenen Korrekturfaktor, sodass sich für diesen Betriebszeitraum eine korrigierte Zeitdauer $t_x$ ergibt, die sich nach der folgenden Formel berechnet:

$$t_X = t_s + t_s\left(\frac{I_S - I_{Max}}{I_S}\right)(d_X - d_s)/(d_{Max} - d_s)$$

Diese Berechnungsformel ist in der Steuervorrichtung hinterlegt und findet Anwendung, wenn sich die Betriebsdauer für den wenigstens einen UV-Strahler der UV-Bestrahlungsvorrichtung im Zeitbereich zwischen Sollbetriebsdauer und maximaler Betriebsdauer befindet. Wird in der UV-Bestrahlungsvorrichtung mehr als nur ein UV-Strahler verwendet, ist es sinnvoll, gleichartige Strahler einzusetzen und diese zum gleichen Zeitpunkt in Betrieb zu nehmen, sodass für alle Strahler der UV-Bestrahlungsvorrichtung gleiche Bestrahlungszeiten zur Anwendung kommen können.

Wie bereits erwähnt, ist es zweckmäßig, nach Erreichen der maximalen Betriebsdauer keine weitere Verlängerung der Zeitdauer vorzunehmen, sondern die Zeitverlängerung bei der maximalen Betriebsdauer beizubehalten. Im Zeitpunkt $d_{Max}$ reduziert sich der Korrekturfaktor auf den Term $(I_S - I_{Max})/ I_S$, sodass sich die Zeitdauer für die Phase nach Erreichen der maximalen Betriebsdauer wie folgt errechnen lässt:

$$t_X = t_S + t_S \left( \frac{I_S - I_{Max}}{I_S} \right)$$

Anhand der verschiedenen Funktionen für die unterschiedlichen Betriebsphasen steuert die Steuervorrichtung die UV-Bestrahlungsvorrichtung und veranlasst den Betrieb des mindestens einen UV-Strahlers in Abhängigkeit von dessen Betriebsdauer für die jeweils errechnete Zeitdauer.

Die Erfindung kann grundsätzlich bei jedem Laborgerät angewendet werden, in dem bereits bisher eine UV-Bestrahlungsvorrichtung zur Desinfektion eines Arbeits- oder Lagerraums oder sonstiger Bereiche des Laborgeräts zum Einsatz gekommen ist. Abgesehen von Änderungen in der Steuervorrichtung sind ansonsten keine Änderungen oder Umbauten im Laborgerät erforderlich. Nachfolgend werden ein bevorzugtes Laborgerät und bevorzugtes Vorgehen beispielhaft beschrieben. Bei der Einrichtung eines Desinfektionsverfahrens für das Laborgerät geht der Benutzer in bekannter Weise vor und legt wie im Stand der Technik üblich einen geeigneten Bestrahlungs-Zeitraum unter Berücksichtigung der Gegebenheiten des Laborgeräts, der Art und Anzahl der zu verwendenden UV-Strahler sowie der Kontamination fest. Diese der (Standard-) Zeitdauer $t_S$ entsprechende Dauer des Desinfektionsvorgang wird in üblicher Weise in einem Speicher der Steuervorrichtung abgespeichert. In analoger Weise kann die Standard-Bestrahlungsdauer des Desinfektionsvorgangs auch bereits vom Hersteller oder einer mit der Aufstellung und/oder Wartung betrauten Serviceperson ermittelt und abgespeichert werden. Zudem kann in einem Speicher der Steuervorrichtung ein Zeitplan hinterlegt sein, der vorgibt, zu welchen Zeiten Desinfektionsvorgänge gestartet werden sollen. Diese Desinfektionsvorgänge können entweder automatisch von der Steuervorrichtung gestartet werden, oder die Steuervorrichtung gibt eine Meldung aus, beispielsweise durch Anzeige auf einem Display des Laborgeräts, die den Benutzer darauf hinweist, dass ein Desinfektionsvorgang gestartet werden sollte, und der Benutzer löst daraufhin den Desinfektionsvorgang aus. Zu diesem Zweck ist in der Steuervorrichtung ein Zeitmesser vorhanden. Der Zeitmesser gibt zweckmäßig Datum und Uhrzeit vor, nach denen die Steuervorrichtung entsprechend dem hinterlegten Zeitplan Meldungen zu fälligen Desinfektionsvorgängen ausgeben und/oder diese gegebenenfalls selbst auslösen kann.

Im erfindungsgemäßen Laborgerät ist die Steuervorrichtung zudem so ausgebildet, dass mittels des Zeitmessers die Betriebszeit des wenigstens einen UV-Strahlers in der UV-Bestrahlungsvorrichtung des Laborgeräts ermittelt und aufgezeichnet wird. Entsprechend dieser vom Zeitmesser ermittelten und in einem Speicher der Steuervorrichtung hinterlegten Betriebszeit wird in der Steuervorrichtung mithilfe eines Vergleichers ermittelt, ob der wenigstens eine UV-Strahler die in einem Speicher der Steuervorrichtung für diesen Strahler hinterlegte Sollbetriebsdauer $d_S$ bereits erreicht hat oder nicht. Ist dies nicht der Fall, wird der Desinfektionsvorgang mit einer Zeitdauer betrieben, die der hinterlegten (Standard-) Zeitdauer $t_S$ entspricht. Die Steuervorrichtung gibt die Zeitdauer mittels eines Zeitgebers (Timers) entsprechend vor.

Stellt der Vergleicher der Steuervorrichtung jedoch fest, dass die hinterlegte Sollbetriebsdauer $d_S$ für den UV-Strahler erreicht oder bereits überschritten ist, ermittelt ein Prozessor der Steuervorrichtung anhand der im Speicher hinterlegten Korrekturfunktion eine korrigierte Zeitdauer $t_x$ für den vom Zeitmesser ermittelten Zeitpunkt X. Die hinterlegte Korrekturfunktion ist für den verwendeten UV-Strahler typisch und ergibt sich, wie vorstehend beschrieben, insbesondere aus der für diesen Strahlertyp charakteristischen Abnahme der UV-Bestrahlungsstärke mit der Zeit. Die entsprechenden Werte können am einfachsten aus dem vom Hersteller für diesen Typ UV-Strahler zur Verfügung gestellten Datenblatt entnommen oder alternativ in an sich bekannter Weise experimentell ermittelt werden. Dabei ist es nicht erforderlich, für jeden einzelnen verwendeten UV-Strahler eine eigene Korrekturfunktion festzulegen, sondern es kann ein und dieselbe Korrekturfunktion für verschiedene UV-Strahler des gleichen Typs verwendet werden. Entsprechend ist es bevorzugt, beim Einsatz von mehreren UV-Strahlern für die Desinfektionsvorgänge eines erfindungsgemäßen Laborgeräts Strahler des gleichen Typs einzusetzen. Die Steuervorrichtung steuert nun entweder automatisch ausgelöst oder nach Auslösung durch einen Benutzer den Desinfektionsvorgang unter Verwendung des Zeitgebers für die Zeitdauer $t_x$, die gegenüber der (Standard-) Zeitdauer $t_S$ verlängert ist.

Die Verlängerung der Zeitdauer des Desinfektionsvorgangs entsprechend der hinterlegten Korrekturfunktion erfolgt während eines vom Zeitmesser ermittelten Intervalls von der Sollbetriebsdauer $d_S$ des wenigstens einen UV-Strahlers bis zur maximalen Betriebsdauer $d_{Max}$. Die Zeitdauer des Desinfektionsvorgangs wird dabei, vorzugsweise linear, für die ermittelte UV-Strahler-Betriebsdauer in Richtung auf die maximale Betriebsdauer $d_{Max}$ hin verlängert. Auf diese Weise wird erreicht, dass die erforderliche UV-Strahlendosis weiterhin abgegeben wird, obwohl die vorgegebene Sollbetriebsdauer des UV-Strahlers bereits überschritten ist und dieser in Laborgeräten des Standes der Technik bereits hätte ausgetauscht werden müssen. Nach Erreichen der maximalen Betriebsdauer $d_{Max}$ ist auch im erfindungsgemäßen Laborgerät eine ausreichende UV-Desinfektion nicht mehr sichergestellt, und der wenigstens eine UV-Strahler sollte ausgewechselt werden. Hat der Zeitmesser also einen Wert an die Steuervorrichtung übermittelt, der ein Überschreiten der maximalen Betriebsdauer anzeigt, gibt diese eine Meldung an den Benutzer aus, dass der wenigstens eine UV-Strahler ausgetauscht werden muss. Aus Sicherheitsgründen wird jedoch die Zeitdauer des Desinfektionsvorgangs so lange mit der zuvor ermittelten maximalen Dauer fortgeführt, bis der Benutzer den oder die UV-Strahler tatsächlich ausgewechselt hat. Der Auswechselvorgang kann entweder mittels geeigneter Sensoren automatisch festgestellt werden oder dadurch, dass der Benutzer die erfolgte Auswechslung an einer mit der Steuervorrichtung gekoppelten Eingabevorrichtung quittiert.

Nach dem Austausch des wenigstens einen UV-Strahlers beginnt der nächste Desinfektionsvorgang wieder mit der (Standard-) Zeitdauer $t_S$, und das vorstehend beschriebene Verfahren wird wiederholt. Wird anstelle des wenigstens einen zuvor verwendeten UV-Strahlers ein anderer Typ von UV-Strahler eingesetzt, muss auch die Korrekturfunktion entsprechend auf diesen anderen Typ von Strahler angepasst werden. Grundsätzlich können im Laborgerät bereits von Anfang an verschiedene Korrekturfunktionen für unterschiedliche geeignete Typen von UV-Strahlern hinterlegt sein. Die korrekte Korrekturfunktion muss entweder vom Benutzer entsprechend dem von ihm eingesetzten Strahlertyp ausgewählt werden, wozu zweckmäßig eine mit der Steuervorrichtung verbundene Anzeige- und Eingabevorrichtung dient. Alternativ ist der Einsatz "intelligenter" Sockel möglich, die erkennen, welcher Typ von Strahler in sie eingesetzt wurde, und eine entsprechende Meldung an die Steuervorrichtung übermitteln können, die von letzterer verarbeitet und der weiteren Steuerung zugrunde gelegt wird. Eine andere Möglichkeit besteht darin, das Laborgerät - beispielsweise durch Verwendung eines Strahler-spezifischen Sockels - so auszubilden, dass lediglich eine einzige Art von UV-Strahlern im Laborgerät verwendet werden kann, sodass ausschließlich eine einzige Korrekturfunktion zum Einsatz kommen muss.

Die Erfindung soll nachfolgend anhand von Zeichnungen näher erläutert werden. Die Zeichnungen dienen lediglich der Erläuterung einiger bevorzugter Ausführungsbeispiele, auf die die Erfindung jedoch nicht beschränkt ist. Die Figuren sind schematisch. Gleiche Bezugszeichen bezeichnen gleiche Teile, wobei nicht immer sämtliche Teile mit einem Bezugszeichen versehen sind. Im Einzelnen zeigen:

Fig. 1    eine perspektivische Ansicht eines erfindungsgemäßen Laborgeräts am Beispiel einer Sicherheitswerkbank;

Fig. 2    ein Diagramm für vier verschiedene UV-Strahler, in dem deren Lebensdauer gegen die UV-C-Bestrahlungsstärke aufgetragen ist;

Fig. 3    ein Diagramm, in dem für die Werte der Lampe 1 aus Figur 2 die Betriebsstunden gegen die Abnahme der Bestrahlungsstärke aufgetragen sind, und

Fig. 4    das Diagramm der Figur 3 als Linearfunktion vereinfacht.

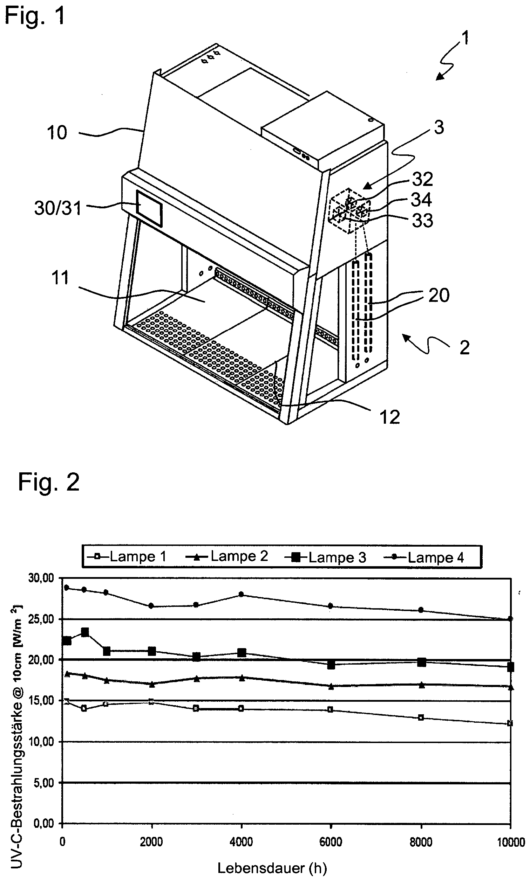

Figur 1 zeigt ein erfindungsgemäßes Laborgerät 1 am Beispiel einer Sicherheitswerkbank. Diese weist ein Gehäuse 10 auf, welches einen Arbeitsraum 11 umgibt, welcher für einen Benutzer über eine Frontöffnung 12, die von einer verschiebbaren Scheibe verschlossen werden kann, zugänglich ist. Die Sicherheitswerkbank dient beispielsweise zur Bearbeitung von biologischen oder mikrobiologischen Proben. Diese können zu einer Kontamination des Arbeitsraums 11 führen. Um eventuell in den Arbeitsraum gelangte Keime wie Bakterien, Viren, Spuren oder Hefen abtöten zu können, ist in der Sicherheitswerkbank eine UV-Bestrahlungsvorrichtung 2 vorgesehen. Diese umfasst im gezeigten Beispiel zwei UV-Strahler 20, die an einer Seitenwand des Gehäuses 10 auf der Seite zum Arbeitsraum 11 hin angeordnet sind. Zur Steuerung des Betriebs der UV-Bestrahlungsvorrichtung 2 ist eine Steuervorrichtung 3 vorhanden, die mit den Strahler 20 verbunden ist.

Desinfektionsvorgänge können von Zeit zu Zeit vom Benutzer der Sicherheitswerkbank ausgelöst werden, indem über eine Eingabevorrichtung 30 (hier ein Touchscreen) ein entsprechender Befehl in die Steuervorrichtung 3 eingegeben wird. Die Steuervorrichtung kann auch so eingerichtet sein, dass sie den Benutzer in bestimmten Zeitabständen daran erinnert, dass ein Desinfektionsvorgang durchgeführt werden sollte. Beispielsweise wird eine entsprechende Meldung auf einem Display 31 (hier in Form eines Touchscreens) oder einer ähnlichen Anzeigevorrichtung angezeigt. Löst der Benutzer durch entsprechende Eingabe einen Desinfektionsvorgang aus, so kann dieser unmittelbar im Anschluss an den Befehl oder auch zeitlich versetzt zu diesem dadurch durchgeführt werden, dass die UV-Strahler 20 für eine vorgegebene Zeitdauer in Betrieb gesetzt werden. Für die Einhaltung der Zeitdauer sorgt ein in die Steuervorrichtung 3 integrierter Zeitgeber 32. Während des Betriebs geben die UV-Strahler 20 UV-Strahlung, insbesondere UV-C-Strahlung mit einer Wellenlänge von 254 nm, in den Arbeitsraum 11 der Sicherheitswerkbank 1 ab.

Die Zeitdauer für den Desinfektionsvorgang zu Beginn der Betriebsdauer der UV-Strahler, von denen nachfolgend davon ausgegangen wird, dass es sich um die gleiche Art von Strahlern mit der gleichen Betriebsdauer handelt, wird vom Benutzer in Abhängigkeit von der Art der abzutötenden Keime im Arbeitsraum 11 vorgegeben und in einem Speicher 33 der Steuervorrichtung 3 hinterlegt. Grundsätzlich wäre es aber auch möglich, von Seiten des Herstellers in der Steuervorrichtung eine bestimmte Anfangs-Zeitdauer vorzugeben, die gegebenenfalls vom Benutzer durch eine eigene Eingabe überschrieben werden kann. Diese Anfangs-Zeitdauer ist bis zu einer Sollbetriebsdauer der UV-Strahler gültig und wird nachfolgend mit $t_S$ bezeichnet.

Die Sollbetriebsdauer ist von der Art der verwendeten UV-Strahler abhängig und bezeichnet diejenige Betriebsdauer, innerhalb derer UV-Strahler die gewünschte Bestrahlungsstärke erzeugen, insbesondere die Nenn-Bestrahlungsstärke, oder nur um ein geringes Maß von dieser abweichen. Die Sollbestrahlungsstärke kann unter anderem aus Daten ermittelt werden, die üblicherweise vom Lampenhersteller zur Verfügung gestellt werden. Alternativ kann die Sollbetriebsdauer durch eigene Messungen festgelegt werden. Figur 2 zeigt ein Beispiel, bei dem die Sollbetriebsdauer anhand eines Lebensdauer-Diagramms des Lampenherstellers ermittelt wird. In Figur 2 ist die Lebensdauer in Stunden für vier verschiedene UV-Strahler, die als Lampe 1 bis Lampe 4 bezeichnet sind, gegen die UV-C-Bestrahlungsstärke, jeweils in einem Abstand von 10 cm zur bestrahlten Fläche, angegeben. Das Diagramm verdeutlicht also für jede Lampe die Änderung der Bestrahlungsstärke mit zunehmender Betriebsdauer. Wie erkennbar, nimmt die Bestrahlungsstärke für jede der Lampen ab einer bestimmten Lebensdauer kontinuierlich ab. Ab diesem Zeitpunkt ist eine Verlängerung der Bestrahlungszeit sinnvoll, um einen Ausgleich für diese Abnahme an Bestrahlungsstärke zu schaffen und eine ausreichende Desinfektion sicherzustellen. Die Sollbetriebsdauer wird auf einen entsprechenden Wert festgesetzt.

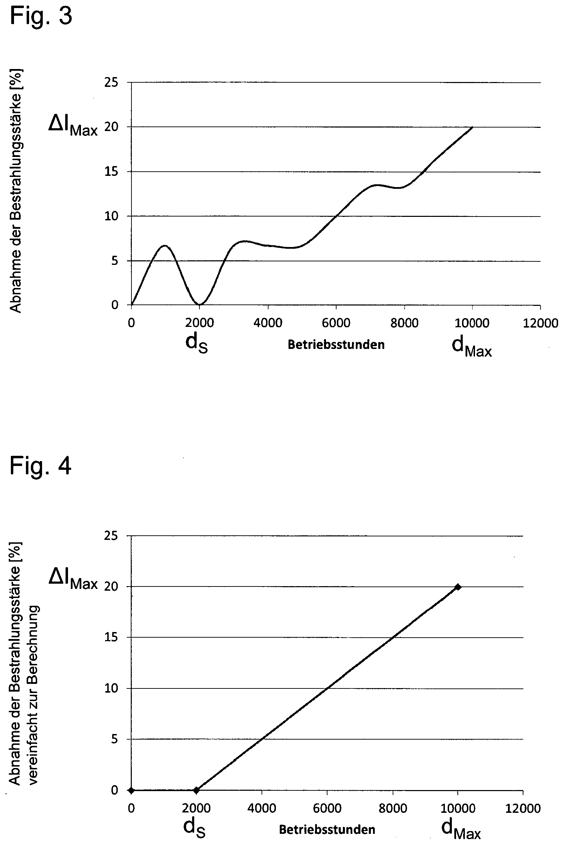

Nachfolgend soll das weitere Vorgehen am Beispiel der Lampe 1 näher erläutert werden. Der Verlauf der Bestrahlungsstärke in Bezug auf die Lebensdauer der Lampe, wie er in Figur 2 dargestellt ist, wird in Figur 3 als Abnahme der Bestrahlungsstärke in Prozent über die Betriebsstunden (= Lebensdauer bzw. Betriebsdauer) wiedergegeben. Wie sich der Kurve entnehmen lässt, benötigt der UV-Strahler (Lampe 1) zunächst eine Einlaufphase, in der die Bestrahlungsstärke zunächst abnimmt, bis nach etwa 2000 Betriebsstunden die maximale Bestrahlungsstärke erreicht ist. Ab diesem Zeitpunkt nimmt die Bestrahlungsstärke immer weiter ab, bis sie bei einer Betriebsdauer von 10.000 Stunden um 20 % gegenüber der maximalen Bestrahlungsstärke reduziert ist. Ab diesem Zeitpunkt ist es nicht mehr sinnvoll, den UV-Strahler weiterhin zu betreiben. Eine Betriebsdauer von 10.000 Stunden wird daher als maximale Betriebsdauer $d_{Max}$ der Lampe 1 definiert. Die Sollbetriebsdauer $d_S$ wird auf 2000 Betriebsstunden festgesetzt.

Gemäß der bevorzugten Ausführungsvariante der Erfindung, die hier beschrieben werden soll, erfolgt die Verlängerung der Zeitdauer zur Kompensation des Verlusts an Bestrahlungsstärke im Zeitraum zwischen der Sollbetriebsdauer und der maximalen Betriebsdauer. Grundsätzlich könnte versucht werden, den in Figur 3 wiedergegebenen Kurvenverlauf als Funktion für die Korrektur der Zeitdauer zu verwenden. Dies würde jedoch erhöhten Aufwand bedeuten, und eine solche Genauigkeit bei der Berechnung der Zeitkorrektur ist in der Regel nicht erforderlich. Aus diesem Grund ist es erfindungsgemäß bevorzugt, den Verlauf der Abnahme der Bestrahlungsstärke bezüglich der Betriebsdauer vereinfacht in einer linearen Funktion darzustellen.

Diese vereinfachte Beziehung ist in Figur 4 grafisch wiedergegeben. Wie ersichtlich, weist die Funktion zunächst während einer Betriebsdauer von 0 bis 2000 Stunden einen waagerechten Abschnitt auf. In diesem Zeitraum erfolgt keine Korrektur der Zeitdauer der Bestrahlung; die Zeitdauer entspricht der vorgegebenen Zeitdauer $t_S$. Bei einer Betriebsdauer von über 2000 Stunden bis hin zur maximalen Betriebsdauer von 10.000 Stunden erfolgt dagegen eine Verlängerung der Zeitdauer, ausgehend von der ursprünglichen Zeitdauer $t_S$. Für jede beliebige Betriebsdauer $d_X$ im Zeitraum $d_S$ bis $d_{Max}$ ergibt sich aus der in Figur 4 dargestellten Beziehung eine zugehörige Abnahme der Bestrahlungsstärke, die durch eine entsprechende Verlängerung der Zeitdauer kompensiert werden soll. Der entsprechende Korrekturfaktor lässt sich rechnerisch angeben als

$$\left(\frac{I_S - I_{Max}}{I_S}\right)(d_X - d_s)/(d_{Max} - d_s)$$

wobei $I_S$ die Bestrahlungsstärke bei der Sollbetriebsdauer ist und $I_{Max}$ die Bestrahlungsstärke bei der maximalen Betriebsdauer. Die Differenz zwischen $I_S$ und $I_{Max}$ ist in Figuren 3 und 4 als $\Delta I_{Max}$ angegeben und beträgt im Beispiel 20 %, woraus sich ein Multiplikator von 0,2 ergibt. Damit ergibt sich für den Betriebszeitraum $d_S$ bis $d_{Max}$ eine Zeitdauer $t_x$, die sich nach der folgenden Formel berechnet:

$$t_X = t_S + t_S\left(\frac{I_S - I_{Max}}{I_S}\right)(d_X - d_s)/(d_{Max} - d_s)$$

Anhand dieser Berechnungsformel berechnet die Steuervorrichtung die Zeitdauer, für welche die UV-Strahler betrieben werden, wenn ihre Betriebsdauer im Zeitraum zwischen der Sollbetriebsdauer und der maximalen Betriebsdauer liegt. Die aktuelle Betriebsdauer der UV-Strahler wird dabei mittels eines Zeitmessers 34 ermittelt, der in die Steuervorrichtung 3 integriert ist.

Nach Erreichen der maximalen Betriebsdauer sollten die UV-Strahler ausgetauscht werden. Um dies zu gewährleisten, kann die Steuervorrichtung 3 so ausgebildet sein, dass sie jeden weiteren Start eines Desinfektionsvorgangs blockiert, bis neue UV-Strahler in die UV-Bestrahlungsvorrichtung eingesetzt wurden. Allerdings besteht in diesem Fall die Gefahr, dass die Sicherheitswerkbank ohne Desinfektion weiterverwendet wird, wodurch sich eine Gefährdung der bearbeiteten Proben und der Benutzer ergeben kann. Aus diesem Grund kann es sinnvoll sein, Desinfektionsvorgänge auch nach Ablauf der maximalen Betriebsdauer der UV-Strahler weiter zuzulassen. In diesem Fall ist es ebenfalls sinnvoll, eine Kompensation für den Verlust an Bestrahlungsstärke weiterhin vorzusehen. In einer Variante der Erfindung erfolgt die Verlängerung der Zeitdauer daher auch nach Überschreiten der maximalen Dauer, und zwar mit dem gleichen Korrekturfaktor, der sich für die maximale Betriebsdauer ergibt. An diesem Punkt gilt für die Zeitdauer die Formel

$$t_X = t_S + t_S \left( \frac{I_S - I_{Max}}{I_S} \right)$$

was im konkreten Beispiel eine Verlängerung um 0,2 $t_S$ bedeutet.

Dem Benutzer wird das Erreichen der maximalen Betriebsdauer im Display 31 des Geräts (hier als Touchscreen an der Gehäusefrontseite) angezeigt, und er wird aufgefordert, die UV-Strahler 20 auszutauschen. Auf dem Display können zudem weitere Informationen zum Desinfektionsvorgang, zum Beispiel, ob bereits eine Zeitverlängerung zum Einsatz kommt, und zum Status der UV-Strahler angezeigt werden. Die Steuervorrichtung 3 kann außerdem so programmiert sein, dass dem Benutzer Eingriffe und Korrekturen in den Verfahrensablauf ermöglicht werden, wie zum Beispiel die bereits erwähnte Eingabe oder Änderung der (Standard-) Zeitdauer $t_S$ oder der korrigierten Zeitdauer $t_x$.

Die Erfindung stellt auf die beschriebene Weise sicher, dass eine zuverlässige Desinfektion erfolgt, ohne dass dies einen frühzeitigen Austausch der UV-Strahler, einen erhöhten Energieverbrauch oder zusätzlichen Aufwand für den Benutzer des Laborgeräts bedeutet.

**Patentansprüche**

1. Laborgerät (1), in dem biologische oder mikrobiologische Proben bearbeitet oder gelagert werden, umfassend eine UV-Bestrahlungsvorrichtung (2) mit mindestens einem UV-Strahler (20) zur Durchführung eines Desinfektionsvorgangs sowie eine Steuervorrichtung (3),
   **dadurch gekennzeichnet,**
   **dass** die Steuervorrichtung (3) zur Steuerung des UV-Strahlers (20) ausgebildet ist und ausgebildet ist, eine Zeitdauer (t) vorzugeben, während welcher der UV-Strahler (20) während des Desinfektionsvorgangs betrieben wird, und die Zeitdauer (t) unter Verwendung einer in der Steuervorrichtung hinterlegten Steuerfunktion in Abhängigkeit von der Betriebsdauer (d) des UV-Strahlers (20) so vorzugeben, dass die Zeitdauer (t) mit zunehmender Betriebsdauer (d) zunimmt.

2. Laborgerät nach Anspruch 1,
   **dadurch gekennzeichnet,**
   **dass** in der Steuervorrichtung (3) eine Sollbetriebsdauer ($d_S$) hinterlegt ist, die insbesondere einer Betriebsdauer entspricht, bei der die Bestrahlungsstärke (I) gerade noch einer Soll-Bestrahlungsstärke ($I_S$) und insbesondere der Nenn-Bestrahlungsstärke des mindestens einen UV-Strahlers (20) entspricht, und die Steuervorrichtung (3) ausgebildet ist, die Verlängerung der Zeitdauer (t) ab dieser Sollbetriebsdauer ($d_S$) vorzunehmen.

3. Laborgerät nach Anspruch 2,
   **dadurch gekennzeichnet,**
   **dass** in der Steuervorrichtung (3) eine maximale Betriebsdauer ($d_{Max}$) hinterlegt ist, die insbesondere der vom Hersteller des mindestens einen UV-Strahlers (20) definierten maximalen Lebensdauer entspricht, und die Steuervorrichtung ausgebildet ist, die Verlängerung der Zeitdauer (t) im Zeitraum zwischen der Sollbetriebsdauer ($d_S$) und der maximalen Betriebsdauer ($d_{Max}$) vorzunehmen.

4. Laborgerät nach einem der vorhergehenden Ansprüche,
   **dadurch gekennzeichnet,**

**dass** die Verlängerung der Zeitdauer (t) in Abhängigkeit von der Betriebsdauer (d) einer Linearfunktion folgt.

5. Laborgerät nach Anspruch 4,
**dadurch gekennzeichnet,**
**dass** die Linearfunktion dadurch definiert ist, dass die Bestrahlungsstärke ($I_S$) bei der Sollbetriebsdauer ($d_S$) als 100 % festgelegt, die Bestrahlungsstärke ($I_{Max}$) bei der maximalen Betriebsdauer ($d_{Max}$) als x % (< 100 %) ermittelt und zwischen beiden Eckpunkten eine lineare Abnahme der Bestrahlungsstärke angenommen wird, wobei sich für die jeweilige Betriebsdauer (d) im Bereich zwischen Sollbetriebsdauer ($d_S$) und maximaler Betriebsdauer ($d_{Max}$) aus dieser Linearfunktion ein Korrekturfaktor für die Verlängerung der Zeitdauer (t) ergibt.

6. Laborgerät nach Anspruch 5,
**dadurch gekennzeichnet,**
**dass** die Zeitdauer (t) zu einem Betriebszeitpunkt (x), der im Zeitraum zwischen Sollbetriebsdauer ($d_S$) und maximaler Betriebsdauer ($d_{Max}$) liegt, durch die Formel

$$t_X = t_S + t_S \left( \frac{I_S - I_{Max}}{I_S} \right) (d_X - d_s)/(d_{Max} - d_s)$$

definiert ist, wobei $t_S$ eine für den Desinfektionsvorgang festgelegte Zeitdauer darstellt, wenn der mindestens eine UV-Strahler (20) die Sollbetriebsdauer ($d_S$) noch nicht erreicht hat.

7. Laborgerät nach Anspruch 5,
**dadurch gekennzeichnet,**
**dass** die Linearfunktion, anstelle der durch die beiden Eckpunkte definierten Geraden, durch eine Ausgleichsgerade definiert ist, die aus Messwerten der Bestrahlungsstärke im Bereich zwischen Sollbetriebsdauer ($d_S$) und maximaler Betriebsdauer ($d_{Max}$) ermittelt ist.

8. Laborgerät nach einem der vorhergehenden Ansprüche,
**dadurch gekennzeichnet,**
**dass** es sich um einen Klimaschrank, insbesondere einen Inkubator, einen Trockenschrank oder einen Laborkühlschrank, einen Laborabzug oder eine Sicherheitswerkbank handelt.

9. Verfahren zum Betrieb eines Laborgeräts (1) nach einem der vorhergehenden Ansprüche,
**dadurch gekennzeichnet,**
**dass** die Steuervorrichtung (3) unter Verwendung einer in der Steuervorrichtung hinterlegten Steuerfunktion eine Zeitdauer (t) in Abhängigkeit von der Betriebsdauer (d) des UV-Strahlers (20) ermittelt, wobei die Zeitdauer (t) mit zunehmender Betriebsdauer (d) zunimmt, und die UV-Bestrahlungsvorrichtung (2) für die ermittelte Zeitdauer (t) einen Desinfektionsvorgang durchführt.

10. Verfahren nach Anspruch 9,
**dadurch gekennzeichnet,**
**dass** in der Steuervorrichtung (3) eine Sollbetriebsdauer ($d_S$) hinterlegt ist, bis zu deren Erreichen keine Verlängerung der Zeitdauer (t) erfolgt.

11. Verfahren nach Anspruch 9,
**dadurch gekennzeichnet,**
**dass** die UV-Bestrahlungsvorrichtung (2) nach Überschreiten der maximalen Betriebsdauer ($d_{Max}$) Desinfektionsvorgänge mit der gleichen Zeitdauer (t) durchführt, wie sie für den Zeitpunkt der maximalen Betriebsdauer ($d_{Max}$) errechnet wurde, insbesondere mit einer Zeit entsprechend der Formel

$$t_X = t_S + t_S \left( \frac{I_S - I_{Max}}{I_S} \right)$$

12. Verfahren nach einem der Ansprüche 9 bis 11,
dadurch geke n nzeich net,

dass bei Erreichen der maximalen Betriebsdauer eine entsprechende Warnung, insbesondere in Form eines optischen und/oder akustischen Signals, an den Benutzer ausgegeben wird.

**Claims**

1. Laboratory apparatus (1) in which biological or microbiological samples are processed or stored, comprising a UV irradiation device (2) having at least one UV emitter (20) for carrying out a disinfection process, and a control device (3), **characterized in that**
the control device (3) is designed to control the UV emitter (20) and is designed to specify a duration (t) during which the UV emitter (20) is operated during the disinfection process, and to specify the duration (t), using a control function stored in the control device, as a function of the operating time (d) of the UV emitter (20) such that the duration (t) increases as the operating time (d) increases.

2. Laboratory apparatus according to claim 1,
**characterized in that**
a target operating time ($d_s$) is stored in the control device (3), which target operating time in particular corresponds to an operating time in which the irradiance (I) substantially corresponds to a target irradiance ($I_s$) and in particular corresponds to the nominal irradiance of the at least one UV emitter (20), and the control device (3) is designed to extend the duration (t) starting from this target operating time ($d_s$).

3. Laboratory apparatus according to claim 2,
**characterized in that**
a maximum operating time ($d_{Max}$) is stored in the control device (3), which maximum operating time in particular corresponds to the maximum service life defined by the manufacturer of the at least one UV emitter (20), and the control device is designed to extend the duration (t) in the period of time between the target operating time ($d_s$) and the maximum operating time ($d_{Max}$).

4. Laboratory apparatus according to any the preceding claims,
**characterized in that**
the extension of the duration (t) as a function of the operating time (d) follows a linear function.

5. Laboratory apparatus according to claim 4,
**characterized in that**
the linear function is defined by the irradiance ($I_s$) at the target operating time ($d_s$) being set as 100%, the irradiance ($I_{Max}$) at the maximum operating time ($d_{Max}$) being determined as x% (<100%), and a linear decrease in the irradiance being assumed between the two vertices, a correction factor for the extension of the duration (t) being obtained from this linear function for the relevant operating time (d) in the range between the target operating time ($d_s$) and the maximum operating time ($d_{Max}$).

6. Laboratory apparatus according to claim 5,
**characterized in that**
the duration (t) at a time of operation (x) which lies in the period of time between the target operating time ($d_s$) and the maximum operating time ($d_{Max}$) is defined by the formula

$$t_x = t_s + t_s \left( \frac{I_s - I_{Max}}{I_s} \right) (d_x - d_s)/(d_{Max} - d_s)$$

where $t_s$ represents a period of time set for the disinfection process if the at least one UV emitter (20) has not yet reached the target operating time ($d_s$).

7. Laboratory apparatus according to claim 5,
**characterized in that**
the linear function, instead of the line defined by the two vertices, is defined by a line of best fit which is determined from measured values of the irradiance in the range between the target operating time ($d_s$) and the maximum operating time ($d_{Max}$).

**8.** Laboratory apparatus according to any of the preceding claims,
**characterized in that**
said laboratory apparatus is a climatic cabinet, in particular an incubator, a drying cabinet or a laboratory refrigerator, a laboratory fume cupboard or a safety cabinet.

**9.** Method for operating a laboratory apparatus (1) according to any of the preceding claims,
**characterized in that**
the control device (3), using a control function stored in the control device, determines a duration (t) as a function of the operating time (d) of the UV emitter (20), the duration (t) increasing as the operating time (d) increases, and the UV irradiation device (2) carries out a disinfection process for the determined duration (t).

**10.** Method according to claim 9,
**characterized in that**
a target operating time ($d_s$) is stored in the control device (3), and the duration (t) is not extended until the target operating time is reached.

**11.** Method according to claim 9,
**characterized in that**
the UV irradiation device (2), after the maximum operating time ($d_{Max}$) has been exceeded, carries out disinfection processes having the same duration (t) as calculated for the point in time of the maximum operating time ($d_{Max}$), in particular with a time corresponding to the formula

$$t_x = t_s + t_s\left(\frac{I_s - I_{Max}}{I_s}\right)$$

**12.** Method according to any of claims 9 to 11,
**characterized in that**
when the maximum operating time is reached, a corresponding warning, in particular in the form of an optical and/or acoustic signal, is output to the user.

**Revendications**

**1.** Appareil de laboratoire (1), dans lequel des échantillons biologiques ou microbiologiques sont traités ou stockés, comprenant un dispositif d'irradiation d'UV (2) comportant au moins un émetteur d'UV (20) permettant d'effectuer un processus de désinfection ainsi qu'un dispositif de commande (3),
**caractérisé en ce**
**que** le dispositif de commande (3) est conçu pour permettre la commande de l'émetteur d'UV (20) et est conçu pour prédéterminer une période de temps (t) pendant laquelle l'émetteur d'UV (20) est actionné pendant le processus de désinfection, et la période de temps (t) est prédéterminée en fonction du temps de fonctionnement (d) de l'émetteur d'UV (20) au moyen d'une fonction de commande mémorisée dans le dispositif de commande, de sorte que la période de temps (t) augmente avec l'augmentation du temps de fonctionnement (d).

**2.** Appareil de laboratoire selon la revendication 1,
**caractérisé en ce**
**qu'**une période de fonctionnement cible ($d_s$) est mémorisée dans le dispositif de commande (3), laquelle période de fonctionnement cible correspond en particulier à une période de fonctionnement pendant laquelle l'intensité d'irradiation (I) avoisine une intensité d'irradiation cible ($I_s$), et en particulier l'intensité d'irradiation nominale de l'au moins un émetteur d'UV (20), et le dispositif de commande (3) est conçu pour prolonger la période de temps (t) à partir de cette période de fonctionnement cible ($d_s$).

**3.** Appareil de laboratoire selon la revendication 2,
**caractérisé en ce**
**qu'**une période de fonctionnement maximale ($d_{Max}$) est mémorisée dans le dispositif de commande (3), laquelle période de fonctionnement maximale correspond en particulier à la durée de vie maximale définie par le fabricant de l'au moins un émetteur d'UV (20), et le dispositif de commande est conçu pour prolonger la période de temps (t) dans l'intervalle de temps entre la période de fonctionnement cible ($d_s$) et la période de fonctionnement maximale

11

($d_{Max}$).

4. Appareil de laboratoire selon l'une des revendications précédentes,
   **caractérisé en ce**
   **que** le prolongement de la période de temps (t) en fonction de la période de fonctionnement (d) suit une fonction linéaire.

5. Appareil de laboratoire selon la revendication 4,
   **caractérisé en ce**
   **que** la fonction linéaire est définie en ce que l'intensité d'irradiation ($I_s$) pour la période de fonctionnement cible ($d_s$) est déterminée comme étant de 100 %, l'intensité d'irradiation ($I_{Max}$) pour la période de fonctionnement maximale ($d_{Max}$) est déterminée comme étant de x % (< 100 %) et une diminution linéaire de l'intensité d'irradiation est supposée entre deux points d'angle, un facteur de correction pour la prolongation de la période de temps (t) résultant de cette fonction linéaire pour la période de fonctionnement (d) respective dans la plage entre la période de fonctionnement cible ($d_s$) et la période de fonctionnement maximale ($d_{Max}$).

6. Appareil de laboratoire selon la revendication 5,
   **caractérisé en ce**
   **que** la période de temps (t) à un moment de fonctionnement (x), lequel se situe dans l'intervalle de temps entre la période de fonctionnement cible ($d_s$) et la période de fonctionnement maximale ($d_{Max}$), est définie par la formule

$$t_x = t_s + t_s \left( \frac{I_s - I_{Max}}{I_s} \right) (d_x - d_s)/(d_{Max} - d_s)$$

où $t_s$ représente une période de temps fixe pour le processus de désinfection lorsque l'au moins un émetteur d'UV (20) n'a pas encore atteint la période de fonctionnement définie ($d_s$).

7. Appareil de laboratoire selon la revendication 5,
   **caractérisé en ce**
   **que** la fonction linéaire, au lieu de par les lignes droites définies par les deux points d'angle, est définie par une ligne droite d'ajustement qui est déterminée à partir des valeurs mesurées de l'intensité d'irradiation dans la plage entre la période de fonctionnement cible ($d_s$) et la période de fonctionnement maximale ($d_{Max}$).

8. Appareil de laboratoire selon l'une des revendications précédentes,
   **caractérisé en ce**
   **qu'**il s'agit d'une armoire de climatisation, en particulier d'un incubateur, d'une armoire de séchage ou d'un réfrigérateur de laboratoire, d'une sorbonne de laboratoire ou d'une hotte de sécurité.

9. Procédé permettant le fonctionnement d'un appareil de laboratoire (1) selon l'une des revendications précédentes,
   **caractérisé en ce**
   **que** le dispositif de commande (3), au moyen d'une fonction de commande mémorisée dans le dispositif de commande, détermine une période de temps (t) en fonction de la période de fonctionnement (d) de l'émetteur d'UV (20), la période de temps (t) augmentant avec une augmentation de la période de fonctionnement (d), et le dispositif d'irradiation d'UV (2) effectue un processus de désinfection pendant la période de temps déterminée (t).

10. Procédé selon la revendication 9,
    **caractérisé en ce**
    **qu'**une période de fonctionnement cible ($d_s$) est mémorisée dans le dispositif de commande (3), la période de temps (t) n'est pas prolongée jusqu'à ce que ladite période de fonctionnement cible est atteinte.

11. Procédé selon la revendication 9,
    **caractérisé en ce**
    **que** le dispositif d'irradiation d'UV (2), après avoir dépassé la période de fonctionnement maximale ($d_{Max}$), effectue des processus de désinfection avec la même période de temps (t) que celle qui a été calculée pour le moment de la période de fonctionnement maximale ($d_{Max}$), en particulier avec un temps selon la formule

$$t_x = t_s + t_s \left( \frac{I_s - I_{Max}}{I_s} \right)$$

**12.** Procédé selon l'une des revendications 9 à 11,
**caractérisé en ce**
**que** lorsque la période de fonctionnement maximale est atteinte, un avertissement correspondant, en particulier sous forme d'un signal optique et/ou acoustique, est délivré à l'utilisateur.

## Fig. 1

1

10

3

32
34
33

30/31

11

20

2

12

## Fig. 2

Lampe 1    Lampe 2    Lampe 3    Lampe 4

UV-C-Bestrahlungsstärke @ 10cm [W/m²]

30,00

25,00

20,00

15,00

10,00

5,00

0,00

0    2000    4000    6000    8000    10000

Lebensdauer (h)

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2009218512 A1
- GB 2551349 A
- US 20150060696 A1
- US 20080199353 A1
- EP 3269798 A1